# EUROPÄISCHE PATENTSCHRIFT

(11) **EP 4 017 430 B1**
(45) Veröffentlichungstag und Bekanntmachung des Hinweises auf die Patenterteilung: **14.02.2024**
(21) Anmeldenummer: 20758211.5
(22) Anmeldetag: 19.08.2020
(51) Int. Cl.: A61F 5/01

(54) **ORTHESE ZUR ERZEUGUNG EINER SUPINATION UND/ODER PRONATION**
ORTHOSIS FOR CREATING A SUPINATION AND/OR PRONATION
ORTHÈSE POUR LA PRODUCTION D'UNE SUPINATION ET/OU D'UNE PRONATION

(30) Priorität: 20.08.2019 DE 102019212410
(43) Veröffentlichungstag der Anmeldung: 29.06.2022
(73) Patentinhaber: Pohlig GmbH, 83278 Traunstein (DE)
(72) Erfinder: SCHÄFER, Michael, 83278 Traunstein (DE); KIENZLE, Christian, 83064 Raubling (DE); DUMBERGER, Christian, 83379 Wonneberg (DE); HOFMANN, Sebastian, 83329 Waging (DE)
(74) Vertreter: Pfenning, Meinig & Partner mbB
(86) Internationale Anmeldenummer: PCT/EP2020/073235
(87) Internationale Veröffentlichungsnummer: WO 2021/032795

(56) Entgegenhaltungen:
- EP-A1- 2 874 579
- EP-B1- 2 874 579
- WO-A1-99/37257
- DE-T2- 69 616 629
- DE-T2- 69 616 629
- US-A- 5 385 536
- US-A1- 2015 073 323

## Beschreibung

Die Erfindung betrifft eine Orthese mit einer Unterarmaufnahme zur Anlage an einen Unterarm eines Patienten und einer Rumpfauflage zur Anlage an die dem Unterarm zugeordneten Seite des Rumpfes des Patienten, welche der Erzeugung einer Supination und/oder Pronation dient. Die US 2015/073323 A1 offenbart den Oberbegriff des Anspruchs 1.

Es gibt unterschiedliche Diagnosen, bei denen es gewünscht ist, mittels einer Orthese in der Schulter eine Außenrotation und in Hand- bzw. Unterarm des Patienten eine Supination oder Pronation zu erzeugen. Ein Beispiel hierfür ist die kindliche Plexusparese. Typischerweise werden dabei zur Behandlung mehr oder minder starre Pflaster/Bandagen, Verbände um Unterarm und/oder Oberarm und/oder Hand angelegt oder auch Orthesen welche die Hand bzw. den Unterarm in Einheit mit dem Oberarm in definierter Position, also jeweils der Supination oder Pronation, halten sollen.

Die bisherigen Ansätze haben eine Reihe von Nachteilen, so sind sie beispielsweise großvolumig, nur einmal verwendbar, nicht oder nur schwer im Verlauf der Behandlung anpassbar und im Alltag hinderlich oder nur begrenzt wirksam.

Vor allem der schlechte Tragekomfort wird bei derartigen Versorgungslösungen oftmals bemängelt.

Entsprechend stellt sich die Aufgabe, eine verbesserte Orthese, insbesondere für die beschriebenen Indikationen, bereitzustellen.

Diese Aufgabe wird durch die Gegenstände der unabhängigen Patentansprüche gelöst. Vorteilhafte Ausführungsformen ergeben sich aus den abhängigen Patentansprüchen, der Beschreibung und den Figuren.

Ein Aspekt betrifft eine Orthese mit einer Unterarmaufnahme zur Anlage an oder Aufnahme von einem Unterarm und bevorzugt auch an die dem Unterarm zugeordnete Hand eines Patienten, sowie mit einer Rumpfauflage zur Anlage an die dem Unterarm zugeordnete Seite des Rumpfes des Patienten. Dabei ist die Unterarmaufnahme mit der Rumpfauflage zur Erzeugung einer Supination des Unterarms oder, äquivalent, der dem Unterarm zugeordneten Hand zur der Erhaltung der Supination und/oder Pronation zumindest in einer Raumrichtung mit einer vorgegebenen Kraft, aber in dieser Richtung verstellbar, beispielsweise verschiebbar, direkt verbunden. Unter "direkt verbunden" kann hier verstanden werden, dass die Unterarmaufnahme mit der Rumpfauflage verbunden ist, ohne dass sich in einer Kraftübertragungskette zwischen Unterarmaufnahme und Rumpfauflage eine mit Unterarmaufnahme und/oder Rumpfauflage verbundene weitere Komponente, welche zur Anlage an einen weiteren Körperteil wie beispielsweise den Oberarm oder die Schulter oder die Achsel ausgebildet ist. Die vorgegebene Kraft kann durch ein entsprechendes Bauteil unveränderlich vorgegeben oder definiert sein und somit eine starre Verbindung realisieren, oder aber durch ein anderes entsprechendes Bauteil, beispielweise eine Gasdruckfeder und/oder einen Seilzug, dynamisch vorgegeben oder definiert sein und somit eine elastische Verbindung realisieren. In beiden Fällen kann die vorgegebene Kraft insbesondere auch eine einstellbare Kraft sein, beispielsweise über eine Einstellmöglichkeit an dem jeweiligen entsprechenden Bauteil. Die Rumpfauflage kann beispielsweise in Form eines Beckenkorbs ausgeführt sein oder einen solchen umfassen.

So kann mit der Orthese im Schultergelenk eine Position des Oberarms in der Transversalachse, also Extension bzw. Flexion, sowie eine Position des Oberarms in der Sagittalachse, also Abduktion bzw. Adduktion, starr vorgegeben sein, während gleichzeitig eine Position des Oberarms in der Longitudinalachse wahlweise starr und/oder frei beweglich und/oder dynamisch beweglich mit einer vorgebbaren oder vorgegeben Kraft oder einem vorgebbaren oder vorgegebenen Moment beaufschlagt und/oder verstellbar starr (in unterschiedlichen Positionen feststellbar) und/oder limitierbar (über einen einstellbaren eingeschränkten Bereich verstellbar) beweglich vorgegeben sein kann. Im Ellenbogengelenk kann eine Position des Unterarmes mit der Orthese hinsichtlich der Beugung bzw. Streckung des Unterarmes wahlweise starr und/oder frei beweglich und/oder verstellbar starr (in unterschiedlichen Positionen feststellbar) und/oder limitierbar (über einen einstellbaren eingeschränkten Bereich verstellbar) und/oder hinsichtlich der Supination bzw. Pronation wahlweise starr und/oder frei beweglich und/oder dynamisch beweglich mit einer vorgebbaren oder vorgegeben Kraft oder einem vorgebbaren oder vorgegebenen Moment beaufschlagt und/oder verstellbar starr (in unterschiedlichen Positionen feststellbar) und/oder limitierbar (über einen einstellbaren eingeschränkten Bereich verstellbar) beweglich vorgegeben sein. Das Handgelenk kann dabei starr gehalten sein.

Bevorzugt ist die Unterarmaufnahme dabei in einer Raumebene starr oder elastisch, aber in dieser Raumebene verstellbar verbunden, insbesondere ohne dabei in der zu der Raumebene senkrecht stehenden Raumrichtung verstellbar zu sein. Die Unterarmaufnahme kann also in einer Raumrichtung, welche bevorzugt einer Längs- oder Hochachse des Rumpfes des Patienten entspricht, zumindest in Teilen, starr oder elastisch, aber verstellbar mit der Rumpfauflage verbunden sein. Dabei kann die Orthese zum Erhalten der Supination und der Pronation geeignet sein, und dann in der jeweiligen Anwendung auf entweder die Supination oder die Pronation eingestellt werden. Dabei gelten die im Folgenden für die Supination beschriebenen Vorteile und vorteilhaften Ausführungsformen analog zur Supination entsprechend auch für die Pronation. Folglich offenbart beispielsweise der folgende Absatz zur gegenüber der Rumpfauflage zur Einstellung der Supination verstellbaren Unterarmaufnahme auch die gegenüber der Rumpfauflage zur Einstellung der Pronation verstellbare Unterarmaufnahme.

Die Unterarmaufnahme ist somit gegenüber der Rumpfauflage zur Einstellung der Supination verstellbar. Insbesondere kann die Rumpfauflage zur Einstellung der Supination diskret, also in vorgegebenen diskreten Schritten, verstellbar sein. Die diskreten Schritte können dabei durch die Gestaltung der Orthese und somit unveränderlich vorgegeben sein. Alternativ oder ergänzend kann die Rumpfauflage eine Anlagefläche aufweisen, an welcher die Unterarmaufnahme in unterschiedlichen Positionen befestigt werden kann, beispielsweise magnetisch und/oder mit einem Klettverschluss. Hier und im Folgenden kann unter dem Begriff "Supination" die Supination von Hand und/oder Unterarm inklusive einer gewünschten Haltung beteiligter Gliedmaßen, wie beispielsweise dem Oberarm, relativ zu Unterarm und/oder Rumpf, insbesondere Schulter verstanden werden.

Dadurch, dass die Unterarmaufnahmen mit der Rumpfauflage bezüglich der Supinationsstellung starr verbunden ist, ist bei bestimmungsgemäßem, d. h. alltäglichem Gebrauch der Orthese durch den Patienten die Stellung in gewünschter Weise durch die Orthese vorgegeben, welche bedingt durch die Starrheit der Verbindung den Unterarm des Patienten in der gewünschten Haltung relativ zum Rumpf hält oder stützt. Dadurch, dass die Unterarmaufnahme verstellbar mit der Rumpfauflage verbunden ist, ist bei bestimmungsgemäßer Anpassung der Orthese an den Patienten, beispielsweise durch einen Arzt oder Orthopäden durch das Verstellen die durch die Orthese vorgegebene Stellung in gewünschter Weise vorgebbar und an die Anatomie des Patienten sowie eine Verletzung oder Einschränkung des Patienten oder ein Behandlungsziel anpassbar. Die Unterarmaufnahme kann auch lösbar starr verbunden sein, insbesondere werkzeugfrei lösbar starr verbunden, sodass durch Lösen der Verbindung zwischen Unterarmaufnahme und Rumpfauflage der Nutzer zeitweise, z.B. im Alltag, die Beweglichkeit einschränken und zeitweise, z.B. kurzzeitig für eine besondere Aufgabe, die Beweglichkeit entschränken kann. Die Unterarmaufnahme kann dabei, wie weiter unten beschrieben, in Längs- und/oder Rotationsachse von Unterarm und/oder Oberarm (insbesondere passiv oder dynamisch) verstellbar sein.

Das hat den Vorteil, dass die Supination besonders stabil und zuverlässig genau in der für den jeweiligen Patienten sinnvollen Haltung durch die Orthese vorgegeben werden kann, wobei die geschilderte Konfiguration der Orthese nicht nur eine mehrfache Verwendung der Orthese bei unterschiedlichen Patienten ermöglicht, sondern auch die Anpassung der Orthese an eine Veränderung, beispielsweise schrittweise Genesung des Patienten. Überdies kann so auch eine Beeinträchtigung des Patienten im Alltag durch die Orthese verringert werden, was wiederum die Akzeptanz erhöht und den Behandlungserfolg steigert.

Die Unterarmaufnahme ist gegenüber der Rumpfauflage zur Erzeugung einer maximalen Supination (und/oder Pronation, wie aus der oben erläuterten Äquivalenz von Bezügen auf die Supination zur Pronation hervorgeht; verstellbar. Die Unterarmaufnahme ist gegenüber der Rumpfauflage also über einen so großen Bereich verstellbar, dass nach allgemeiner Erfahrung nicht die Orthese das Ausmaß der erzeugenden Supination limitiert, sondern die Beweglichkeit des Patienten in Hand, Unterarm, und ggf. Oberarm.

Das hat den Vorteil, dass die Orthese besonders flexibel eingesetzt werden kann, insbesondere für unterschiedliche Diagnosen, unterschiedliche Behandlungsverläufe und unterschiedliche Patienten.

In einer besonders vorteilhaften Ausführungsform ist vorgesehen, dass die Orthese auch eine Schulteraufnahme zur Anlage an die den Unterarm und damit auch der Hand zugeordneten Schulter des Patienten aufweist, welche mit der Unterarmaufnahme und/oder der Rumpfauflage verbunden ist. Dabei ist die Unterarmaufnahme gegenüber der Schulteraufnahme und der Rumpfauflage zur Erzeugung der Supination, insbesondere der maximalen Supination, der Hand verstellbar. Insbesondere kann die Unterarmaufnahme zur Erzeugung der Supination oder Pronation der Hand (17) und einer Schulterrotation verstellbar sein. Die Erzeugung der Schulterrotation kann dabei mit der Erzeugung der Supination gekoppelt sein. Die Schulteraufnahme kann beispielsweise durch entsprechende Bänder mit Unterarmaufnahmen und/oder Rumpfauflage verbunden sein.

Das hat den Vorteil, dass Rumpfauflage und/oder Unterarmaufnahme gegen ein Verrutschen gesichert sind, sowie allgemein auch die Position von Hand und Unterarm, und damit die Supination genauer definiert werden können, da mit Unterarm, Rumpf und Schulter drei Fixpunkte im Raum gegeben sind und somit die Anzahl unerwünschter Freiheitsgrade reduziert und der durch die Orthese vermittelte Halt verbessert wird.

Dabei kann vorgesehen sein, dass die Schulteraufnahme zumindest in Teilen, also teilweise oder ganz, einstellbar und/oder zumindest in (insbesondere anderen) Teilen, also teilweise oder ganz, starr mit der Unterarmaufnahme und/oder der Rumpfauflage verbunden ist.

Beispielsweise kann die Schulteraufnahme mit der Unterarmaufnahme und/oder der Rumpfauflage durch entsprechende Kunststoffschienen verbunden sein.

Die Verstellbarkeit führt hier zu der bereits oben erläuterten Flexibilität der Orthese, wobei die Starrheit eine genauere Vorgabe der Supination incl. der Haltung der verbundenen Körperteile erlaubt.

Die Unterarmaufnahme weist eine (erste) Gelenkeinheit mit einer quer oder senkrecht zur Längsachse des Unterarms und/oder zur Längsachse des zum Unterarm zugehörigen Oberarms verlaufenden Rotations- oder Schwenkachse auf. Die Gelenkeinheit hier ist ein Scharniergelenk. Die Schwenkachse der Gelenkeinheit oder des Scharniergelenks verläuft dabei durch den Ellenbogen, idealerweise durch die Schwenk- oder Gelenkachse des Ellenbogens des Patienten. Bei bestimmungsgemäßem Gebrauch kann eine Bewegung um die Schwenkachse der ersten Gelenkeinheit somit einem Beugen und/oder Strecken des Unterarms gegenüber dem Oberarm entsprechen. Dieses Beugen und/oder Strecken ist dann insbesondere unabhängig von anderen Arm- und Handbewegungen.

Das hat den Vorteil, dass der Komfort im Alltag für den Patienten verbessert bzw. die Behinderung des Patienten durch die Orthese verringert wird, so dass diese im Alltag länger getragen werden kann und somit einen verbesserten Behandlungserfolg erzielt.

Dabei kann vorgesehen sein, dass die Gelenkeinheit ausgebildet ist, ein in der und unter Beibehaltung der Supinationsstellung des Unterarms geführtes Beugen des dem Unterarm zugeordneten Ellenbogens um zumindest 45° zuzulassen, bevorzugt zumindest 60° und besonders bevorzugt zumindest 90°. Das hat den Vorteil, dass die Orthese den Patienten besonders wenig behindert und so den im letzten Absatz beschriebenen Effekt verstärkt.

In einer anderen vorteilhaften Ausführungsform ist vorgesehen, dass die Unterarmaufnahme eine andere (zweite) Gelenkeinheit mit einer parallel zur Längs- oder Hochachse des Rumpfes des Patienten und/oder der Längsachse des durch die Orthese gehaltenen Oberarms des Patienten verlaufenden Rotations- oder Schwenkachse aufweist. Bevorzugt ist die andere Gelenkeinheit dabei zwischen der ersten Gelenkeinheit und der Rumpfauflage angeordnet, verbindet also insbesondere die erste Gelenkeinheit mit der Rumpfauflage. Bei bestimmungsgemäßem Gebrauch kann eine Bewegung um die Schwenkachse der zweiten Gelenkeinheit somit einer Rotation des Oberarms im Schultergelenk entsprechen. Bei festgehaltener Position des Ellenbogens führt diese Rotation des Oberarms zum Bewegen der Hand auf einem Kreisbogensegment zum Rumpf des Patienten hin oder vom Rumpf fort. Die Rotation ist dann insbesondere unabhängig von dem Beugen und/oder Strecken des Unterarms gegenüber dem Oberarm und/oder von anderen Arm- und Handbewegungen.

Das hat den Vorteil, dass die Beweglichkeit des Arms in der Schulter bei Beibehalten der Supinationsstellung weitgehend beibehalten wird. Durch die körpernahe Anordnung der anderen Gelenkeinheit wird dieser Vorteil verstärkt.

In einer besonders vorteilhaften Ausführungsform ist vorgesehen, dass die Unterarmaufnahme zumindest in Teilen, also teilweise oder ganz, gegenüber der Schulteraufnahme und/oder gegenüber der Rumpfauflage um die Längsachse des Unterarms drehbar verstellbar ist, um den Grad der Supination der Hand zu verstellen, also nach Bedarf variabel einzustellen, insbesondere zu maximieren. Dabei kann die Unterarmaufnahme als Ganzes oder teilweise, d. h. Teile der Unterarmaufnahme wie beispielsweise eine Schale für den Unterarm gegenüber der Schulteraufnahme und/oder gegenüber der Rumpfauflage drehbar verstellbar sein. Bei bestimmungsgemäßem Gebrauch kann eine Drehung um die Längsachse somit einer Rotation der Hand gegenüber dem Ellenbogen entsprechen. Die Drehung ist dann insbesondere unabhängig von der Rotation des Oberarms in der Schulter und/oder von dem Beugen und/oder Strecken des Unterarms gegenüber dem Oberarm und/oder von anderen Arm- und Handbewegungen.

Das hat den Vorteil, dass die gleiche Orthese für unterschiedliche gewünschte Supinationsgrade sowie für verschiedene Patienten und auch für den gleichen Patienten in unterschiedlichen Phasen der Behandlung genutzt werden kann. Durch die individuelle Anpassbarkeit der Orthese werden wiederum Akzeptanz und Behandlungserfolg verbessert.

In einer weiteren vorteilhaften Ausführungsform ist vorgesehen, dass die Unterarmaufnahme (zumindest in Teilen) gegenüber der Schulteraufnahme und/oder der Rumpfauflage um das Schultergelenk der dem Unterarm zugeordneten Schulter ventral/dorsal verdrehbar verstellbar ist, d. h. in ventrale und/oder dorsale Richtung verstellbar ist. Auch kann die Unterarmaufnahme (zumindest in Teilen) gegenüber der Schulteraufnahme und/oder der Rumpfauflage in Richtung der Längsachse der Unterarmaufnahme (oder entsprechend des Unterarms) und/oder in Richtung einer Rotation um die Längsachse der Unterarmaufnahme (oder entsprechend des Unterarms) verstellbar sein. Alternativ oder ergänzend kann die Unterarmaufnahme (zumindest in Teilen) gegenüber der Schulteraufnahme und/oder der Rumpfauflage in Richtung der Längsachse des zugeordneten Oberarms und/oder in Richtung einer Rotation um die Längsachse des zugeordneten Oberarms verstellbar sein.

Auch diese Art der Flexibilität vergrößert das mögliche Einsatzspektrum der Orthese und erhöht Akzeptanz und Erfolgswahrscheinlichkeit der Behandlung.

In einer anderen vorteilhaften Ausführungsform ist vorgesehen, dass die Unterarmaufnahme zumindest in Teilen gegenüber der Schulteraufnahme und/oder der Rumpfauflage in Längsrichtung des Unterarms verschiebbar verstellbar ist.

Das hat ebenfalls den Vorteil, dass unterschiedliche Anatomien bestmöglich durch die Orthese berücksichtigt werden können und somit Akzeptanz und Erfolg der Behandlung verbessert wird.

In einer besonders vorteilhaften Ausführungsform ist vorgesehen, dass die Orthese eine Koppelvorrichtung aufweist, beispielsweise eine Rastvorrichtung, durch welche die Unterarmaufnahme mit der Rumpfauflage wiederholt und zerstörungsfrei lösbar, insbesondere werkzeugfrei lösbar, verbindbar ist. Bevorzugt kann dies über eine Rastverbindung erfolgen.

Das hat den Vorteil, dass im alltäglichen Gebrauch nicht nur die Position von Unterarm gegenüber dem Rumpf leicht verstellt und damit an die gegebenen Bedingungen angepasst werden kann, sondern auch für spezifische Handlungen, beispielsweise Zähneputzen, Unterarmaufnahme und Rumpfauflage vorübergehend entkoppelt, also voneinander getrennt werden können. Auch dies erhöht eine Akzeptanz der Orthese und verbessert damit die Erfolgswahrscheinlichkeit der Behandlung.

In einer weiteren vorteilhaften Ausführungsform ist vorgesehen, dass die Rumpfauflage als Beckenauflage ausgebildet ist, insbesondere als Beckenring.

Das hat den Vorteil, dass die Rumpfauflage besonders gut am Körper fixiert ist und die Rumpfauflage als Referenzpunkt für die Unterarmaufnahme besonders gut definiert ist, wodurch die Orthese genauer an die gewünschte Supination angepasst werden kann.

In einer weiteren vorteilhaften Ausführungsform ist vorgesehen, dass die Rumpfauflage, insbesondere auch die Schulteraufnahme und/oder die Unterarmaufnahme, ausgebildet ist, den zum Unterarm gehörenden Oberarm zumindest abschnittsweise am Rumpf anliegend zu halten. Beispielsweise kann die Rumpfauflage und/oder Schulteraufnahme und/oder Unterarmaufnahme so ausgebildet sein, dass der Oberarm in einem Achselbereich an den Rumpf anliegt, d. h. beispielsweise ein Winkel zwischen Oberarm und Rumpf weniger als 45° oder weniger als 20° beträgt. Bevorzugt kann dabei die Rumpfauflage und/oder Schulterauflage und/oder Unterarmaufnahme ausgebildet sein, den Unterarm über seine volle Länge am Rumpf anliegend zu halten.

Das hat den Vorteil, dass der Unterarm und damit die Hand besonders stabil in einer gewünschten Haltung positioniert werden kann, d. h. die Supination besonders genau vorgegeben werden kann. Überdies ist die genannte Haltung des Oberarms auch besonders wenig anstrengend bzw. angenehm und unauffällig im Alltag, so dass wiederum Akzeptanz und Behandlungserfolg verbessert werden.

Ein weiterer Aspekt betrifft auch ein Verfahren zum Führen und Halten eines Unterarms durch eine Orthese, mit einem Erzeugen einer Supination, insbesondere einer maximalen Supination der dem Unterarm zugeordneten Hand durch ein gegenüber dem Rumpf verstellbares Halten des Unterarms durch die Orthese.

Vorteile und vorteilhafte Ausführungsformen des Verfahrens entsprechen dabei Vorteilen und vorteilhaften Ausführungsformen der Orthese.

Die oben in der Beschreibung, insbesondere auch dem einleitenden Teil der Beschreibung, genannten Merkmale und Merkmalskombinationen sowie die nachfolgend in der Figurenbeschreibung genannten und/ oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch Kombination einzelner aus den erläuterten Ausführungen separierter Merkmale hervorgehen und erzeugbar sind. Es sind auch Ausführungen und Merkmalskombinationen als offenbart anzusehen, die somit nicht alle Merkmale eines ursprünglich formulierten unabhängigen oder abhängigen Anspruchs aufweisen. Es sind darüber hinaus auch Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder von diesen abweichen.

Ausführungsbeispiele der Erfindung werden nachfolgend anhand schematischer Zeichnungen erläutert. Dabei zeigen:
- Fig. 1: eine schematische Ansicht einer ersten beispielhaften Ausführungsform einer Orthese;
- Fig. 2: eine ventrale Ansicht der Orthese aus Fig. 1;
- Fig. 3: eine Schnittdarstellung der Orthese von Fig. 1;
- Fig. 4: eine dorsale Ansicht der Orthese aus Fig. 1 an einem Patienten;
- Fig. 5: die Orthese mit dem Patienten aus Fig. 4 aus einer Schräg-Oben-Ansicht;
- Fig. 6: eine seitliche Schräg-Oben-Ansicht einer weiteren beispielhaften Ausführungsform einer Orthese;
- Fig. 7: eine ventrale Ansicht der Orthese aus Fig. 6 mit einem Patienten;
- Fig. 8: eine schematische Ansicht einer weiteren beispielhaften Ausführungsform einer Orthese;
- Fig. 9: eine dorsale Ansicht einer anderen beispielhaften Ausführungsform einer Orthese;
- Fig. 10: eine seitliche Ansicht der Orthese aus Fig. 9; und
- Fig. 11: eine ventrale Ansicht der Orthese aus Fig. 9.

In den Figuren werden gleiche oder funktionsgleiche Elemente mit den gleichen oder ähnlichen Bezugszeichen versehen.

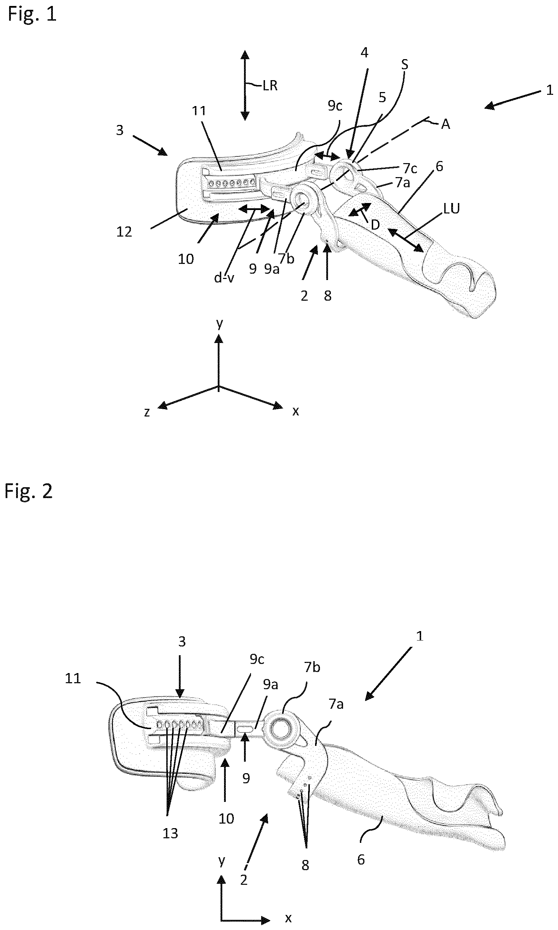

Fig. 1 zeigt eine perspektivische Ansicht einer ersten beispielhaften Ausführungsform einer Orthese. Die Orthese 1 weist dabei eine Unterarmaufnahme 2 zur Anlage an einen Unterarm eines Patienten sowie eine Rumpfauflage 3 zur Anlage an die dem Unterarm zugeordnete Seite des Rumpfes des Patienten auf. Dabei ist die Unterarmaufnahme 2 mit der Rumpfauflage 3 zur Erzeugung einer Supination der dem Unterarm zugeordneten Hand vorliegend starr, aber verstellbar verbunden. Vorliegend weist die Unterarmaufnahme 2 eine Gelenkeinheit 4 mit einem Scharnier 5 auf, mittels welchem sich eine Halteschale 6 der Unterarmaufnahme 2 um eine Schwenkachse A verschwenken lässt. Die Schwenkachse A verläuft dabei quer, d. h. im Wesentlichen senkrecht zur Längsachse LU der Schale 6 und damit eines zugeordneten Unterarms sowie zu einer Längsachse des zugehörigen Oberarms und/oder einer Längs- oder Hochachse LR des nicht gezeigten Rumpfes des Patienten. Vorliegend ist die Gelenkeinheit 4 ausgebildet, ein Beugen des zugeordneten Ellenbogens um mehr als 90° zuzulassen, um die Schwenkachse A.

Dabei ist im gezeigten Beispiel ein erster Scharnierarm 7a, welcher vorliegend bogenförmig ausgebildet ist und an jeweiligen Enden des Bogens um die Schwenkachse drehbar an den zweiten Scharnierarmen 7b, 7c gelagert ist, zwischen den Enden mit derSchale 6 verbunden. Eine Rasteinrichtung 8 ermöglicht dabei ein Verdrehen der Schale 6 um die Längsachse LU, wie es durch den Doppelpfeil D dargestellt ist. Die Unterarmaufnahme 2 ist somit vorliegend zumindest in Teilen gegenüber der Rumpfauflage 3 um die Längsachse des Unterarms drehbar verstellbar befestigt. Die Rasteinrichtung 8 kann dabei auch derart ausgestaltet sein, dass die Schale 6 gegenüber der Rumpfauflage 3 in der Längsrichtung LU verschiebbar verstellbar ist. In diesem Fall ist dann die Unterarmaufnahme 2 zumindest in Teilen gegenüber der Rumpfauflage 3 in Längsrichtung des Unterarms verschiebbar verstellbar befestigt. Alternativ kann auch vorgesehen sein, die Unterarmaufnahme 2 zumindest in Teilen gegenüber der Rumpfauflage 3 in Längsrichtung LU verschiebbar verstellbar zu befestigen mit einer im weiteren Befestigungseinrichtung 9, welche im gezeigten Beispiel zwischen der Gelenkeinheit 4 und der Rumpfauflage 3 angeordnet ist und ein Verschieben, wie es durch den Pfeil S dargestellt ist, ermöglicht. Beispielsweise kann diese weitere Befestigungseinrichtung 9 eine Schraubverbindung umfassen, welche jeweilige Befestigungsarme 9a, 9b an einem Befestigungsfuß 9c in unterschiedlichen Positionen verschrauben lässt. Im gezeigten Beispiel sind die Arme 9a, 9b an den zweiten Scharnierarmen 7b, 7c angebracht oder in diese integriert.

Im gezeigten Beispiel ist der Befestigungsfuß 9c auch Teil einer Koppelvorrichtung 10, welche die Unterarmaufnahme 2 mit der Rumpfauflage 3 lösbar verbindet. Die Koppelvorrichtung 10 ist dabei derart ausgestaltet, dass zwischen Rumpfauflage 3 und Unterarmaufnahme 2 eine Rastverbindung bereitgestellt wird, bei welcher der Befestigungsfuß 9c in einer Führungsschiene 11 in unterschiedlichen Positionen verrastbar ist. Die Schiene 11 ist dabei an einem Polstermodul 12 angebracht, welche vorliegend zur Anlage an ein Becken des Patienten ausgebildet ist.

Die Positionen sind dabei in ventraler oder dorsaler Richtung an unterschiedlichen Orten, insbesondere auf einer Linie, verortet, was ein Verschieben des Befestigungsfußes 9c und damit der Unterarmaufnahme 2 entlang des mit d-v gekennzeichneten Doppelpfeils ermöglicht.

Insgesamt wird so eine höchst flexibel einsetzbare, komfortable und somit den Heilerfolg verbessernde Orthese 1 bereitgestellt.

In Fig. 2 ist die Orthese aus Fig. 1 dargestellt. Besonders gut sind aus dieser Perspektive die unterschiedlichen Rastmöglichkeiten 13 der Koppelvorrichtung 10 zu sehen, sowie die entsprechenden Rastmöglichkeiten der Verbindung 8.

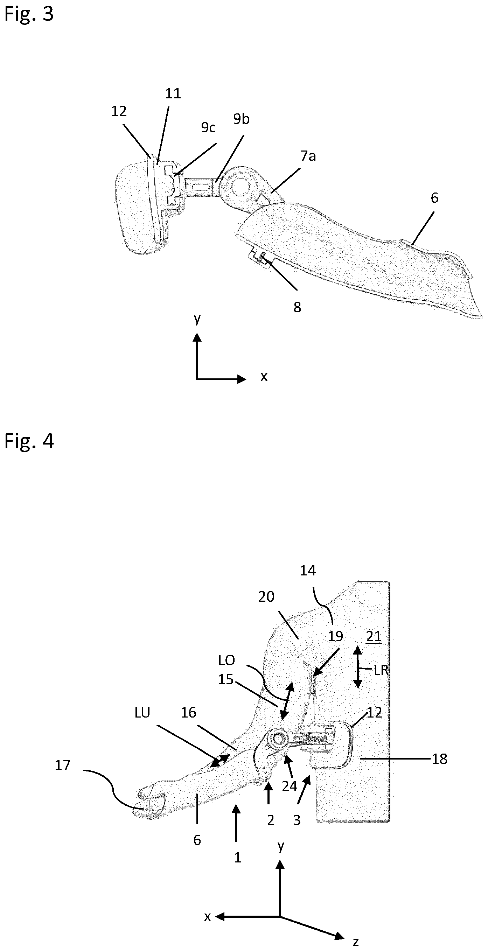

Gerade die Rastmöglichkeiten der Verbindung 8 und die schienenartige Ausgestaltung des Befestigungsfußes 9c und der Schiene 11 sind in Fig. 3 nochmals detailliert dargestellt.

In Fig. 4 ist die Orthese der vorhergehenden Figuren mit einem Patienten 14 dargestellt. Dabei sind die vorliegend der rechten Schulter 20 zugeordneten Oberarm 15 mit Unterarm 16 und Hand 17 von der Orthese 1 in Supination gehalten, wobei die Rumpfauflage mit der Polstereinheit 12 auf einem Becken 18 des Patienten 18 aufliegt. Der Oberarm 15 liegt dabei in dem Bereich 19 an dem Rumpf 21 des Patienten 14 an. Somit wird im gezeigten Beispiel die Supination besonders wirkungsvoll erreicht, da die Hand 17 flexibel und genau in die gewünschte Haltung gebracht wird durch die Orthese 1. Der Ellenbogen 24 wird dabei vorliegend in einer vorgegebenen Position mit festgelegtem Abstand zum Rumpf 21 gehalten.

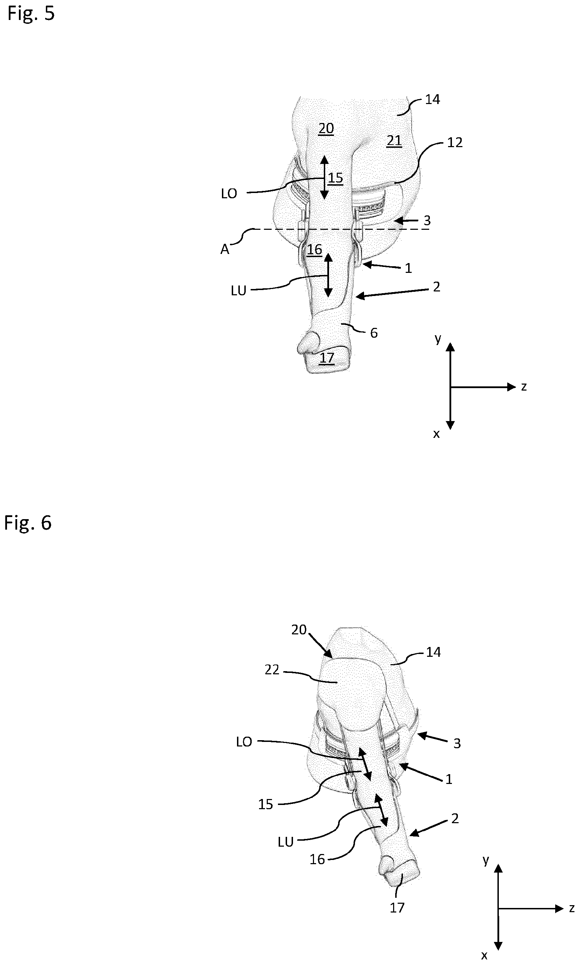

Dies ist auch in Fig. 5 in einer weiteren beispielhaften Haltung des Patienten 14 bzw. des Unterarms 16 mit der Hand 17 relativ zu dem Rumpf 21 dargestellt.

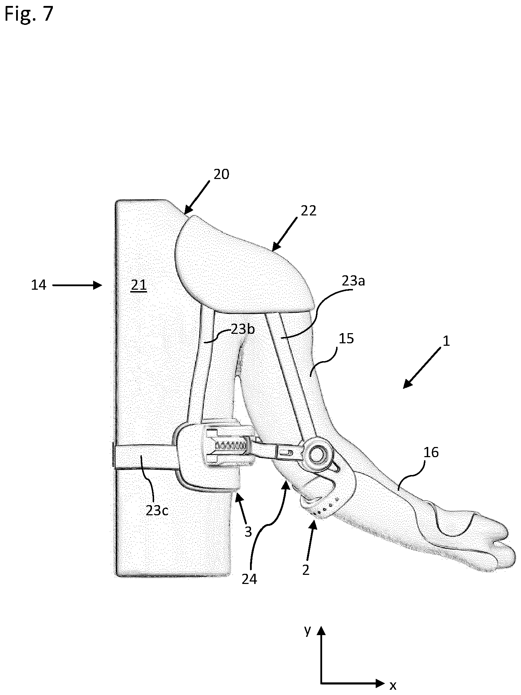

In Fig. 6 ist aus einer ähnlichen Perspektive eine weitere beispielhafte Ausführungsform einer Orthese dargestellt. Die Orthese 1 weist in diesem Fall neben der Rumpfauflage 3 und der Unterarmaufnahme 2 auch eine Schulteraufnahme 22 auf, welche zur Anlage an die dem Unterarm 16 zugeordnete Schulter 20 des Patienten 14 ausgebildet ist. Die Schulteraufnahme 22 ist dabei vorliegend sowohl mit der Unterarmaufnahme 2 und der Rumpfauflage 3 verbunden. Wie in Fig. 7 ersichtlich, ist die Orthese 1 mit der Schulteraufnahme 22 durch entsprechende Bänder 23a, 23b mit der Unterarmaufnahme 2 bzw. der Rumpfauflage 3 verbunden, um Stabilität und Sitz und damit Genauigkeit der Orthese zu verbessern. Im gezeigten Beispiel ist die Rumpfauflage 3 durch ein weiteres Band 23c zur zusätzlichen Stabilisierung als Beckenring ausgebildet.

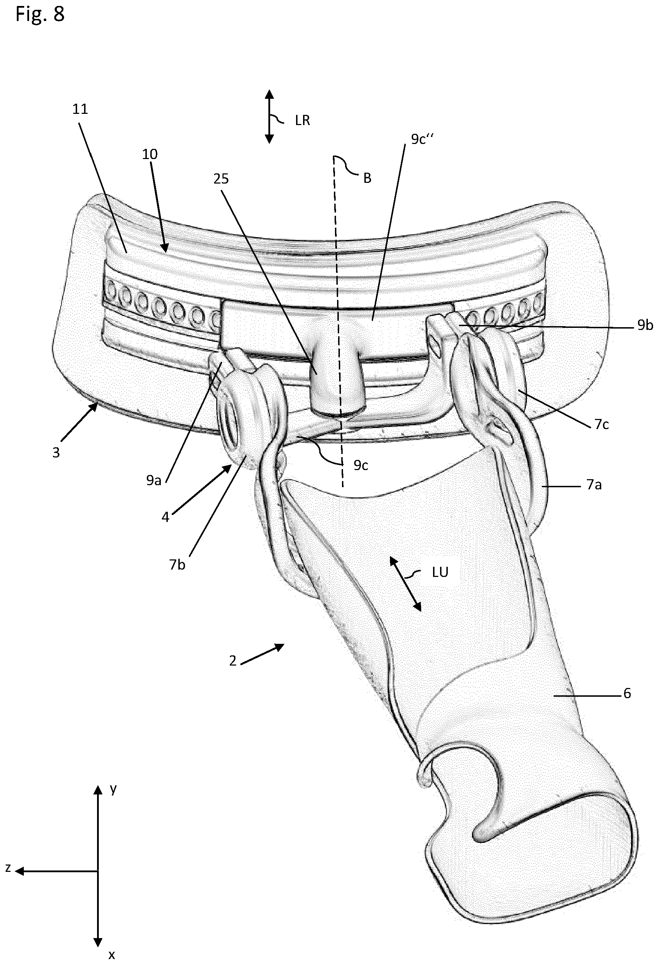

In Fig. 8 ist eine weitere beispielhafte Ausführungsform einer Orthese dargestellt, welche weitgehend der Orthese von Fig. 1 entspricht. Dabei weist die Unterarmaufnahme 2 eine weitere, also hier zweite, Gelenkeinheit 25 auf, die es erlaubt, Teile der Unterarmaufnahme 2, insbesondere die Schale 6, um eine weitere Schwenk- oder Rotationsachse B zu verschwenken. Die Rotationsachse B verläuft dabei vorliegend senkrecht zur Rotationsachse A der ersten Gelenkeinheit 4 und bei bestimmungsgemäßem Gebrauch am Patienten entlang der Längs- oder Hochachse LR dessen Rumpfes 21.

Im gezeigten Beispiel ist die Gelenkeinheit 25 in Koppelvorrichtung 10 und der Befestigungseinrichtung 9. Vorliegend weist nämlich der Befestigungsfuß 9c zwei gegeneinander um die Rotationsachse B verschwenkbare Befestigungsfußteile 9c', 9c" auf, wovon ein Befestigungsfußteil 9c' mit den Befestigungsarmen 9a, 9b verbunden ist und der andere Befestigungsfußteil 9c"in der Führungsschiene 11 in unterschiedlichen Positionen verrastbar gehalten ist.

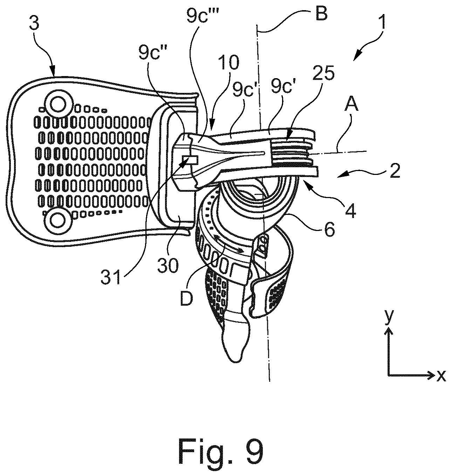

In dem in Fig. 9 gezeigten Ausführungsbeispiels der Orthese 1 ist die Rumpfauflage 3 beispielhaft als Beckenauflage ausgeführt. Die Unterarmaufnahmen 2 ist über die Koppelvorrichtung 10 an der Rumpfauflage 3 befestigt. Dabei entspricht der Führungsschiene 11 vorliegend eine Anlagefläche 30, an welcher der Befestigungsfußteil 9c" als Verbindungsstück an unterschiedlichen Positionen befestigt werden kann. Beispielsweise kann der Befestigungsfußteil 9c" mit einer Klettverbindung an der Anlagefläche 30 befestigt werden. Die Befestigungseinrichtung 9 weist dabei vorliegend noch ein weiteres Befestigungsfußteil 9c‴ auf, welches die anderen beiden Befestigungsfußteile 9c" und 9c' verbindet. Die Verbindung 31 zwischen dem Befestigungsfußteil 9c‴ und dem Befestigungsfußteile 9c" ist dabei werkzeugfrei lösbar und koppelbar ausgeführt, beispielsweise als eine Rastverbindung wie eine Schwalbenschwanzverbindung mit einem einrastenden Schnapper. Dadurch kann das als Verbindungsstück ausgeführte Befestigungsfußteil 9c" in der einmal eingestellten Position an der Anlagefläche 30 verbleiben und kurzfristig die Unterarmaufnahme 2 durch den Patienten von der Rumpfauflage 3 gelöst werden ohne die eingestellte Position zu verändern und damit den Therapieerfolg zu gefährden. Auch können so unterschiedlich dicke Befestigungsfußteile 9c" gewählt werden um eine Abduktion/Adduktion des Schultergelenks vorzugeben. Die Extension/Flexion des schultergelenkt wird über die Position des Befestigungsfußteile 9c" auf der Anlagefläche 30 vorgegeben und kann so individuell eingepasst oder auch nachgepasst werden.

Die Gelenkeinheit 25 lagert vorliegend den Befestigungsfußteil 9c' um die Rotationsachse B schwenk- oder drehbar an dem Befestigungsfußteil 9c‴ und damit auch dem Befestigungsfußteil 9c", da Befestigungsfußteil 9c‴ und Befestigungsfußteil 9c" in verbundenem Zustand eine starre Einheit bilden. Die Rotationsachse fällt hier mit der Longitudinalachse des zugeordneten Schultergelenks zusammen, sodass die Gelenkeinheit 25 ein Schulter-Rotations-Orthesengelenk bildet. Das Schulter-Rotations-Orthesengelenk gibt eine mögliche Au-ßen- bzw. Innenrotation des Schultergelenks vor. Mit dem Schulter-Rotations-Orthesengelenk kann somit eine Position des Oberarms in der Longitudinalachse wahlweise starr und/oder frei beweglich und/oder dynamisch beweglich mit einer vorgebbaren oder vorgegeben Kraft oder einem vorgebbaren oder vorgegebenen Moment beaufschlagt und/oder verstellbar starr (in unterschiedlichen Positionen feststellbar) und/oder limitierbar (über einen einstellbaren eingeschränkten Bereich verstellbar) beweglich vorgegeben werden.

Im gezeigten Beispiel ist der Befestigungsfußteil 9c' als Schlitten ausgeführt, der in dem hier entsprechend als Schiene ausgeführten Befestigungsfußteil 9c‴ fährt. Alternativ kann das Schulter-Rotations-Orthesengelenk auch mit der Gelenkeinheit 25 als (technisch einfachere) Buchsen-Achsen-Konstruktion ausgeführt werden, welche dann idealerweise in Verlängerung des Oberarmes unter dem Ellenbogen angeordnet ist.

Bei einer starren Vorgabe der Position des Oberarms in der Longitudinalachse können Schlitten und Schiene, also Befestigungsfußteil 9c‴ und Befestigungsfußteil 9c', einteilig ausgeführt sein. Bei einer frei beweglichen Vorgabe der Position des Oberarms in der Longitudinalachse kann der Schlitten frei in der Schiene laufen.

Bei einer dynamisch beweglichen mit einer vorgebbaren oder vorgegeben Kraft oder einem vorgebbaren oder vorgegebenen Moment beaufschlagten Vorgabe der Position des Oberarms in der Longitudinalachse können Schlitten und Schiene mit einem internen Federelement, z.B. einem Gummizug und/oder einer Gasdruckfeder und/oder einer Spiralfeder und/oder einem Seilzug, miteinander verbunden sein. Alternativ oder ergänzend kann ein externes Federelement vorgesehen sein, beispielsweise zwischen der Rumpfauflage 3 und der Unterarmaufnahme 2 bzw. dem Befestigungsfußteil 9c'. Dabei kann der Schlitten frei in der Schiene laufen.

Bei einer verstellbar starren Vorgabe der Position des Oberarms in der Longitudinalachse kann die Bewegung des Schlittens in der Schiene mit einem Fixierelement, beispielsweise einem Stift, an einer von mehreren unterschiedlichen Stellen fixiert bzw. blockiert. Die Blockierung kann fix, also insbesondere nicht werkzeugfrei lösbar, oder wieder lösbar, also insbesondere werkzeugfrei lösbar, ausgeführt sein. In letzterem Fall kann das Fixierelement, beispeilsweise der Stift, in anderer (Sperr-)Position fixiert werden um eine andere Position des Oberarms starr vorzugeben.

Bei einer limitierbaren Vorgabe der Position des Oberarms in der Longitudinalachse können in der Schiene an unterschiedlichen Stellen Anschlagelemente angeordnet sein, an welche der Schlitten bei einer Rotation des Oberarms anstößt. Vorteilhafterweise können die Anschlagelemente im Laufe der Therapie versetzt werden, sodass die Vorgabe der Position anpassbar ist. Alternativ oder ergänzend kann ein Federelement wie eine Gasdruckfeder zwischen Schiene und Schlitten bzw. zwischen Rumpfauflage 3 und Unterarmaufnahme 2 derart angeordnet sein, dass das Federelement in der vorgegebenen gewünschten Position des Oberarmes in einer (kraftfreien) Neutralposition ist.

Die unterschiedlichen Vorgaben können auch kombiniert werden, beispielsweise können Schlitten und Schiene mit einer Kraft/einem Moment beaufschlagt, im Bewegungsumfang limitiert und in unterschiedlichen Positionen feststellbar sein.

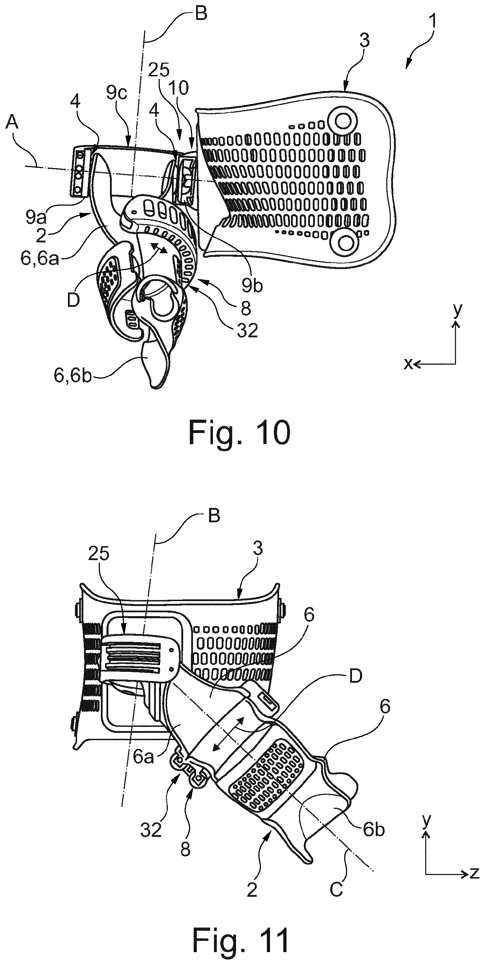

Fig. 10 zeigt, dass die Befestigungsarme 9a, 9b mit dem Befestigungsfußteil 9c Teil der Gelenkeinheit 4 sind, mittels welcher sich die Halteschale 6 der Unterarmaufnahme 2 relativ zu dem Befestigungsfußteil 9c und damit dem Schulter-Rotations-Orthesengelenk um die Schwenkachse A verschwenken lässt. Die Gelenkeinheit 4 bildet dabei vorliegend ein Ellenbogen-Orthesengelenk, welches die Streckung bzw. Beugung des Ellenbogengelenks vorgibt. Die Gelenkeinheit 4 kann die Position des Unterarms hinsichtlich der Beugung bzw. Streckung des Unterarmes wahlweise starr und/oder frei beweglich und/oder verstellbar starr (in unterschiedlichen Positionen feststellbar) und/oder limitierbar (über einen einstellbaren eingeschränkten Bereich verstellbar) vorgeben.

Wie in Fig. 10 und Fig. 11 gezeigt ist die Rasteinrichtung 8 im gezeigten Beispiel Teil einer zusätzlichen Gelenkeinheit 32, mittels welcher Teile der Unterarmaufnahme 2 entsprechend dem Doppelpfeil D, also um eine Rotationsachse C drehbar aneinander gelagert sind. Insbesondere ist durch die zusätzliche Gelenkeinheit 32 ein distaler Abschnitt 6b der Schale 6 gegenüber einem proximalen Abschnitt 6a der Schale 6 drehbar und vorliegend über die Rasteinrichtung 8 in einer vorgegebenen Position fixierbar. Die zusätzliche Gelenkeinheit 32 bildet dabei vorliegend ein Supinations/Pronations-Orthesengelenk, mittels welchem eine Position des Unterarmes hinsichtlich der Supination bzw. Pronation wahlweise starr und/oder frei beweglich und/oder dynamisch beweglich mit einer vorgebbaren oder vorgegeben Kraft oder einem vorgebbaren oder vorgegebenen Moment beaufschlagt und/oder verstellbar starr (in unterschiedlichen Positionen feststellbar) und/oder limitierbar (über einen einstellbaren eingeschränkten Bereich verstellbar) beweglich vorgegeben sein. Dafür ist die zusätzliche Gelenkeinheit 32 im gezeigten Beispiel analog der weiteren Gelenkeinheit 25 als Schlitten-Schiene-System ausgebildet. Entsprechend kann an dem distaler Abschnitt 6b der Schale 6 ein Schlitten der zusätzlichen Gelenkeinheit 32 angeordnet sein und an dem proximalen Abschnitt 6a der Schale 6 eine Schiene der zusätzlichen Gelenkeinheit 32 oder umgekehrt. Die Ausführungen für das Schulter-Rotations-Orthesengelenk gelten entsprechend für das Supinations/Pronations-Orthesengelenk.

## Patentansprüche

1. Orthese (1) mit
- einer Unterarmaufnahme (2) zur Anlage an einen Unterarm (16) eines Patienten (14), und
- einer Rumpfauflage (3) zur Anlage an die dem Unterarm (16) zugeordnete Seite des Rumpfes (21) des Patienten (14),
**dadurch gekennzeichnet, dass**
die Unterarmaufnahme (2) mit der Rumpfauflage (3) zur Erzeugung einer Supination oder einer Pronation der dem Unterarm (16) zugeordneten Hand (17) in zumindest einer Raumrichtung mit einer vorgegebenen Kraft, aber verstellbar, direkt verbunden ist, wobei die Unterarmaufnahme (2) eine Gelenkeinheit (4) mit einem Scharniergelenk und einer quer zur Längsachse (LU) des Unterarms (16) und zur Längsachse (LO) eines zum Unterarm (16) zugehörigen Oberarms (15) verlaufenden Rotationsachse (A) aufweist, welche bei bestimmungsgemä-ßem Gebrauch durch einen zugehörigen Ellenbogen (24) des Patienten (14) verläuft.

2. Orthese (1) nach dem vorhergehenden Anspruch, **dadurch gekennzeichnet, dass** die Unterarmaufnahme (2) starr mit der Rumpfauflage (3) verbunden ist.

3. Orthese (1) nach Anspruch 1, **dadurch gekennzeichnet, dass** die Unterarmaufnahme (2) elastisch mit der Rumpfauflage (3) verbunden ist.

4. Orthese (1) nach einem der vorhergehenden Ansprüche, **dadurch gekennzeichnet, dass** die Unterarmaufnahme (2) gegenüber der Rumpfauflage (3) zur Erzeugung einer maximalen Supination verstellbar ist.

5. Orthese (1) nach einem der vorhergehenden Ansprüche, **gekennzeichnet durch** eine Schulteraufnahme (22) zur Anlage an die dem Unterarm (16) zugeordneten Schulter (20) des Patienten (14), welche mit der Unterarmaufnahme (2) und/oder der Rumpfauflage (3) verbunden ist, wobei die Unterarmaufnahme (2) gegenüber der Schulteraufnahme (22) und der Rumpfauflage (3) zur Erzeugung der maximalen Supination oder Pronation der Hand (17) und insbesondere auch einer Schulterrotation verstellbar ist.

6. Orthese (1) nach dem vorhergehenden Anspruch, **dadurch gekennzeichnet, dass** die Schulteraufnahme (22) verstellbar und/oder starr mit der Unterarmaufnahme (2) und/oder der Rumpfauflage (3) verbunden ist.

7. Orthese (1) nach einem der vorhergehenden Ansprüche, **dadurch gekennzeichnet, dass** die Rotationsachse (A) bei bestimmungsgemäßem Gebrauch durch eine Gelenkachse des zugehörigen Ellenbogens (24) verläuft.

8. Orthese (1) nach einem der vorhergehenden Ansprüche, **dadurch gekennzeichnet, dass** die Gelenkeinheit (4) ausgebildet ist, ein Beugen des zugeordneten Ellenbogens um zumindest 45° zuzulassen, bevorzugt zumindest 60° und besonders bevorzugt zumindest 90°.

9. Orthese (1) nach einem der vorhergehenden Ansprüche, **dadurch gekennzeichnet, dass** die Unterarmaufnahme (2) eine andere Gelenkeinheit (25) mit einer parallel zur Längsachse (LR) des Rumpfes (21) und/oder der Längsachse (LO) des Oberarms (15) verlaufenden Rotationsachse (B) aufweist.

10. Orthese (1) nach einem der vorhergehenden Ansprüche, **dadurch gekennzeichnet, dass** die Unterarmaufnahme (2) zumindest in Teilen gegenüber der Schulteraufnahme (22) und/oder gegenüber der Rumpfauflage (3) um die Längsachse (LU) des Unterarms (16) drehbar verstellbar ist.

11. Orthese (1) nach einem der vorhergehenden Ansprüche, **dadurch gekennzeichnet, dass** die Unterarmaufnahme (2) zumindest in Teilen gegenüber der Schulteraufnahme (22) und/oder der Rumpfauflage (3) um das Schultergelenk ventral/dorsal drehbar verstellbar ist.

12. Orthese (1) nach einem der vorhergehenden Ansprüche, **dadurch gekennzeichnet, dass** die Unterarmaufnahme (2) zumindest in Teilen gegenüber der Schulteraufnahme (22) und/oder der Rumpfauflage (3) in Längsrichtung (LU) des Unterarms (16) verschiebbar verstellbar ist

13. Orthese (1) nach einem der vorhergehenden Ansprüche, **gekennzeichnet durch** eine Koppelvorrichtung (10), durch welche die Unterarmaufnahme (2) mit der Rumpfauflage (3) lösbar verbindbar ist, insbesondere über eine Rastverbindung.

14. Orthese (1) nach einem der vorhergehenden Ansprüche, **dadurch gekennzeichnet, dass** die Rumpfauflage (3) als Beckenauflage ausgebildet ist, insbesondere Beckenring.

15. Orthese (1) nach einem der vorhergehenden Ansprüche, **dadurch gekennzeichnet, dass** die Rumpfauflage (3), insbesondere auch Schulteraufnahme (22) und/oder Unterarmaufnahme, ausgebildet ist, den zum Unterarm (16) gehörigen Oberarm (15) zumindest abschnittsweise, bevorzugt über seine volle Länge, am Rumpf (21) anliegend zu halten.

## Claims

1. An orthosis (1) including:
- a forearm support (2) for contact with a forearm (16) of a patient (14), and
- a torso support (3) for contact with the side of the patient's (14) torso (21) associated with the forearm (16),
**characterized in that**
the forearm support (2) is directly connected to the torso support (3) for producing a supination or a pronation of the hand (17) associated with the forearm (16) in at least one spatial direction with a predetermined force, but adjustably, wherein the forearm support (2) has a joint unit (4) with a hinge joint and an axis of rotation (A) which extends transversely to the longitudinal axis (LU) of the forearm (16) and to the longitudinal axis (LO) of an upper arm (15) associated with the forearm (16) and which, when used as intended, extends through an associated elbow (24) of the patient (14).

2. The orthosis (1) according to the preceding claim, **characterized in that** the forearm support (2) is rigidly connected to the torso support (3).

3. The orthosis (1) according to claim 1, **characterized in that** the forearm support (2) is elastically connected to the torso support (3).

4. The orthosis (1) according to one of the preceding claims, **characterized in that** the forearm support (2) is adjustable relative to the torso support (3) to produce maximum supination.

5. The orthosis (1) according to one of the preceding claims, **characterized by** a shoulder support (22) for contact with the shoulder (20) associated with the forearm (16) of the patient (14), which is connected to the forearm support (2) and/or the torso support (3), wherein the forearm support (2) is adjustable relative to the shoulder support (22) and the torso support (3) to produce the maximum supination or pronation of the hand (17) and in particular also a shoulder rotation.

6. The orthosis (1) according to the preceding claim, **characterized in that** the shoulder support (22) is adjustably and/or rigidly connected to the forearm support (2) and/or the torso support (3).

7. The orthosis (1) according to one of the preceding claims, **characterized in that** the axis of rotation (A) extends through a joint axis of the associated elbow (24) when used as intended.

8. The orthosis (1) according to one of the preceding claims, **characterized in that** the joint unit (4) is designed to permit bending of the associated elbow by at least 45°, preferably at least 60° and particularly preferably at least 90°.

9. The orthosis (1) according to one of the preceding claims, **characterized in that** the forearm support (2) has another joint unit (25) with an axis of rotation (B) running parallel to the longitudinal axis (LR) of the torso (21) and/or the longitudinal axis (LO) of the upper arm (15).

10. The orthosis (1) according to one of the preceding claims, **characterized in that** the forearm support (2) is at least partially adjustable about the longitudinal axis (LU) of the forearm (16) relative to the shoulder support (22) and/or relative to the torso support (3).

11. The orthosis (1) according to one of the preceding claims, **characterized in that** the forearm support (2) can be rotationally adjusted ventrally/dorsally about the shoulder joint at least in parts relative to the shoulder support (22) and/or the torso support (3).

12. The orthosis (1) according to one of the preceding claims, **characterized in that** the forearm support (2) is displaceably adjustable at least in parts relative to the shoulder support (22) and/or the torso support (3) in the longitudinal direction (LU) of the forearm (16).

13. The orthosis (1) according to one of the preceding claims, **characterized by** a coupling device (10), by means of which the forearm support (2) can be detachably connected to the torso support (3), in particular via a latching connection.

14. The orthosis (1) according to one of the preceding claims, **characterized in that** the torso support (3) is designed as a pelvic support, in particular a pelvic ring.

15. The orthosis (1) according to one of the preceding claims, **characterized in that** the torso support (3), in particular also shoulder support (22) and/or forearm support, is designed to hold the upper arm (15) belonging to the forearm (16) in contact with the torso (21) at least in sections, preferably over its full length.

## Revendications

1. Orthèse (1) avec
- un logement d'avant-bras (2) destiné à être appliqué sur un avant-bras (16) d'un patient (14), et
- un support de tronc (3) destiné à être appliqué sur le côté du tronc (21) du patient (14) associé à l'avant-bras (16),
**caractérisée en ce que**
le logement d'avant-bras (2) est relié au support de tronc (3) de manière directe mais réglable pour générer une supination ou une pronation de la main (17) associée à l'avant-bras (16) dans au moins une direction spatiale avec une force prédéfinie, dans laquelle le logement d'avant-bras (2) comporte une unité d'articulation (4) avec une articulation à charnière et un axe de rotation (A) s'étendant transversalement par rapport à l'axe longitudinal (LU) de l'avant-bras (16) et à l'axe longitudinal (LO) d'un bras (15) associé à l'avant-bras (16), lequel passe par un coude (24) associé du patient (14) lors d'une utilisation conforme.

2. Orthèse (1) selon la revendication précédente, **caractérisée en ce que** le logement d'avant-bras (2) est relié de manière rigide au support de tronc (3).

3. Orthèse (1) selon la revendication 1, **caractérisée en ce que** le logement d'avant-bras (2) est relié de manière élastique au support de tronc (3).

4. Orthèse (1) selon l'une quelconque des revendications précédentes, **caractérisée en ce que** le logement d'avant-bras (2) est réglable par rapport au support de tronc (3) pour générer une supination maximale.

5. Orthèse (1) selon l'une quelconque des revendications précédentes, **caractérisée par** un logement d'épaule (22) destiné à être appliqué sur l'épaule (20) du patient (14) associée à l'avant-bras (16), lequel est relié au logement d'avant-bras (2) et/ou au support de tronc (3), dans laquelle le logement d'avant-bras (2) est réglable par rapport au logement d'épaule (22) et au support de tronc (3) pour générer la supination ou la pronation maximale de la main (17) et en particulier aussi une rotation de l'épaule.

6. Orthèse (1) selon la revendication précédente, **caractérisée en ce que** le logement d'épaule (22) est relié de manière réglable et/ou rigide au logement d'avant-bras (2) et/ou au support de tronc (3).

7. Orthèse (1) selon l'une quelconque des revendications précédentes, **caractérisée en ce que** l'axe de rotation (A) passe par un axe d'articulation du coude (24) associé lors d'une utilisation conforme.

8. Orthèse (1) selon l'une quelconque des revendications précédentes, **caractérisée en ce que** l'unité d'articulation (4) est réalisée pour permettre une flexion du coude associé d'au moins 45°, de préférence d'au moins 60° et de manière particulièrement préférée d'au moins 90°.

9. Orthèse (1) selon l'une quelconque des revendications précédentes, **caractérisée en ce que** le logement d'avant-bras (2) comprend une autre unité d'articulation (25) avec un axe de rotation (B) s'étendant parallèlement à l'axe longitudinal (LR) du tronc (21) et/ou à l'axe longitudinal (LO) du bras (15).

10. Orthèse (1) selon l'une quelconque des revendications précédentes, **caractérisée en ce que** le logement d'avant-bras (2) est réglable en rotation autour de l'axe longitudinal (LU) de l'avant-bras (16) au moins en partie par rapport au logement d'épaule (22) et/ou par rapport au support de tronc (3).

11. Orthèse (1) selon l'une quelconque des revendications précédentes, **caractérisée en ce que** le logement d'avant-bras (2) est réglable en rotation ventrale/dorsale autour de l'articulation de l'épaule, au moins en partie, par rapport au logement d'épaule (22) et/ou au support de tronc (3).

12. Orthèse (1) selon l'une quelconque des revendications précédentes, **caractérisée en ce que** le logement d'avant-bras (2) est réglable au moins en partie par rapport au logement d'épaule (22) et/ou au support de tronc (3) dans la direction longitudinale (LU) de l'avant-bras (16).

13. Orthèse (1) selon l'une quelconque des revendications précédentes, **caractérisée par** un dispositif de couplage (10) par lequel le logement d'avant-bras (2) peut être relié de manière libérable au support de tronc (3), en particulier par l'intermédiaire d'une liaison par encliquetage.

14. Orthèse (1) selon l'une quelconque des revendications précédentes, **caractérisée en ce que** le support de tronc (3) est réalisé sous la forme d'un support de bassin, en particulier d'anneau pelvien.

15. Orthèse (1) selon l'une quelconque des revendications précédentes, **caractérisée en ce que** le support de tronc (3), en particulier également le logement d'épaule (22) et/ou logement d'avant-bras, est réalisé pour maintenir le bras (15) appartenant à l'avant-bras (16) appliqué sur le tronc (21) au moins par sections, de préférence sur toute sa longueur.
